# FASCICULE DE BREVET EUROPEEN

(11) **EP 2 800 772 B1**
(45) Date de publication et mention de la délivrance du brevet: **26.10.2016**
(21) Numéro de dépôt: 13701827.1
(22) Date de dépôt: 04.01.2013
(51) Int. Cl.: C08G 69/40, C08L 77/00, A61K 8/88, A61Q 1/00, A61Q 5/00, A61Q 13/00, A61K 8/90, A61K 47/34

(54) **SOLUTION LIQUIDE COMPRENANT DU PEBA**
FLÜSSIGE LÖSUNG MIT PEBA
LIQUID SOLUTION COMPRISING PEBA

(30) Priorité: 05.01.2012 FR 1250119
(43) Date de publication de la demande: 12.11.2014
(73) Titulaire: Arkema France, 92700 Colombes (FR)
(72) Inventeur: MALET, Frédéric, 69007 Lyon (FR); LEMAITRE, Marie-Ange, F-27410 Granchain (FR)
(74) Mandataire: Gavin, Pablo
(86) Numéro de dépôt international: PCT/FR2013/050019
(87) Numéro de publication internationale: WO 2013/102742

(56) Documents cités:
- WO-A1-2012/001298
- CAROL PERKINS GRIFFIN LAI: "HYDROCARBON-TERMINATED POLYETHER-POLYAMIDE BLOCK COPOLYMERS IN PERSONAL CARE AND OTHER PRODUCTS", IP.COM JOURNAL, IP.COM INC., WEST HENRIETTA, NY, US, 14 mai 2003 (2003-05-14), XP013006523, ISSN: 1533-0001

## Description

### Domaine de l'invention

La présente invention a pour objet une composition comprenant un polymère thermoplastique élastomère, en particulier un copolymère à blocs polyéther et blocs polyamide (ci-après PEBA).

La présente invention se rapporte en particulier à une solution liquide, de préférence limpide, comprenant au moins un PEBA, et à l'utilisation de cette solution notamment pour la fabrication de revêtements résistants à l'eau.

### Technique antérieure

Les polymères thermoplastiques se présentent généralement sous forme de granulés ou de poudres. Par conséquent, la formulation avec ces poudres nécessite généralement des étapes intermédiaires de broyage, voire de tamisage, sous forme de poudre fine de D50 généralement inférieure à 10 ou 20 µm, et de dispersion préalable de cette poudre dans un liquide pour que la texture du produit final incorporant la poudre présente une parfaite homogénéité et un aspect uniforme. De plus, la grande volatilité des poudres fines fait que leur utilisation en formulation nécessite beaucoup de précautions. Il est notamment difficile pour les formulateurs de quantifier exactement et de façon reproductible la teneur en poudre des formules.

Parmi les polymères thermoplastiques élastomères, les copolymères à blocs polyéther et blocs polyamide (ci-après PEBA) sont connus pour leur grande souplesse (gamme très étendue), leur élasticité, résistance chimique et tenue à la température, leurs propriétés dynamiques, leurs propriétés stables aux températures de -40°C à 80°C, leurs propriétés imper-respirantes, leur résistance aux UV, etc.

Ces propriétés sont déjà exploitées dans des applications aussi variées que les équipements de sport, les lunettes, le textile, l'automobile, le revêtement de matériaux par des poudres fines de PEBA. En revanche, ces propriétés des PEBAs n'ont jamais été exploitées dans le domaine des formulations liquides, notamment celles utilisées dans les revêtements, la fabrication de films, de peintures, de vernis, de gants, de cosmétiques.

La présente invention a donc pour but de fournir des compositions comprenant du PEBA, prêtes à l'emploi, qui facilitent la mise en oeuvre du PEBA par les formulateurs, en étant directement utilisables (par simple incorporation) dans des formulations de peintures, de revêtement, de cosmétiques par exemple, pour leur conférer des propriétés de rémanence ou de résistance à l'eau. Ainsi, le formulateur n'a plus besoin d'adapter au préalable la forme des PEBAs, vendus dans le commerce sous forme de poudre ou de granulés. De plus, les teneurs en PEBA dans les formulations sont facilement quantifiables et reproductibles.

La présente invention a notamment pour but de fournir de telles compositions de PEBA sous forme liquide prêtes à l'emploi.

La Demanderesse a montré qu'il était possible de fabriquer à partir de PEBA une solution liquide qui reste stable, voire limpide, à température ambiante, c'est-à-dire à une température de 15 à 25°C.

### Description détaillée de l'invention

La présente invention a donc pour objet une composition comprenant : de 1 à 15 parts en poids de PEBA pour 100 parts en poids de solvant, caractérisée en ce que ledit solvant comprend de 1 à 49%, de préférence de 10 à 45%, de préférence de 15 à 40%, de préférence de 20 à 40, en volume d'alcool léger en C1 à C6 et de 51 à 99%, de préférence de 55 à 90%, de préférence de 60 à 85%, de préférence de 60 à 80%, en volume d'un cosolvant miscible avec l'alcool, sur le volume total de solvant, le co-solvant est choisi parmi les acides gras, les esters gras, les alcools gras, les éthers gras et leurs mélanges, et la composition se présente sous la forme d'une solution limpide, présentant à 560 nm une transmission supérieure à 50%, à une température de 15 à 25°C.

Par « miscible » on entend la capacité de liquides à se mélanger. Si le mélange obtenu est homogène, les liquides sont qualifiés de miscibles. Inversement, les liquides sont dits non-miscibles s'ils ne peuvent pas se mélanger et forment un mélange hétérogène : on observe alors plusieurs phases.

Le co-solvant est avantageusement choisi parmi les acides gras, les esters gras, les alcools gras, les éthers gras et leurs mélanges.

Avantageusement, la composition selon l'invention se présente sous la forme d'une solution limpide à température ambiante, soit une température de 15 à 25°C. La notion de limpidité vient en opposition à la notion d'opacité. On peut donc parler de % de transmission de la lumière à une longueur d'onde du visible, typiquement 560 nm. Par « solution limpide » au sens de l'invention on entend une solution présentant à 560 nm une transmission supérieure à 50%, de préférence supérieure à 70%, et par « solution opaque » une solution présentant une transmission inférieure ou égale à 50%, de préférence inférieure ou égale à 70%. De préférence, au sens de la présente invention, le mélange de 2 liquides miscibles forme une solution homogène limpide.

De préférence, la composition comprend de 1 à 10 parts, de préférence de 2 à 8 parts, de préférence de 4 à 6 parts de PEBA pour 100 parts de solvant.

Les « copolymères à blocs polyéther et blocs polyamide » abrégés « PEBA » résultent de la polycondensation de blocs polyamides à extrémités réactives avec des blocs polyéthers à extrémités réactives, telles que, entre autres :
1) blocs polyamides à bouts de chaîne diamines avec des blocs polyoxyalkylènes à bouts de chaînes dicarboxyliques.
2) blocs polyamides à bouts de chaînes dicarboxyliques avec des blocs polyoxyalkylènes à bouts de chaînes diamines, obtenues par cyanoéthylation et hydrogénation de blocs polyoxyalkylène alpha-oméga dihydroxylées aliphatiques appelées polyétherdiols
3) blocs polyamides à bouts de chaînes dicarboxyliques avec des polyétherdiols, les produits obtenus étant, dans ce cas particulier, des polyétheresteramides.

Les blocs polyamides à bouts de chaînes dicarboxyliques proviennent, par exemple, de la condensation de précurseurs de polyamides en présence d'un diacide carboxylique limiteur de chaîne. Les blocs polyamides à bouts de chaînes diamines proviennent par exemple de la condensation de précurseurs de polyamides en présence d'une diamine limiteur de chaîne.

La masse molaire en nombre Mn des blocs polyamides est comprise entre 400 et 20000 g/mole et de préférence entre 500 et 10000 g/mole.

Les polymères à blocs polyamides et blocs polyéthers peuvent aussi comprendre des motifs répartis de façon aléatoire.

On peut utiliser avantageusement trois types de blocs polyamides.

Selon un premier type, les blocs polyamides proviennent de la condensation d'un diacide carboxylique, en particulier ceux ayant de 4 à 20 atomes de carbone, de préférence ceux ayant de 6 à 18 atomes de carbone et d'une diamine aliphatique ou aromatique, en particulier celles ayant de 2 à 20 atomes de carbone, de préférence celles ayant de 6 à 14 atomes de carbone.

A titre d'exemples d'acides dicarboxyliques, on peut citer l'acide 1,4-cyclohexyldicarboxylique, les acides butanedioïque, adipique, azélaïque, subérique, sébacique, dodécanedicarboxylique, octadécanedicarboxylique et les acides téréphtalique et isophtalique, mais aussi les acides gras dimérisés.

A titre d'exemples de diamines, on peut citer la tétraméthylène diamine, l'hexaméthylènediamine, la 1,10-décaméthylènediamine, la dodécaméthylènediamine, la triméthylhexaméthylène diamine, les isomères des bis-(4-aminocyclohexyl)-méthane (BACM), bis-(3-méthyl-4-aminocyclohexyl)méthane (BMACM), et 2-2-bis-(3-méthyl-4-aminocyclohexyl)-propane (BMACP), et para-amino-di-cyclo-hexyl-méthane (PACM), et l'isophoronediamine (IPDA), la 2,6-bis-(aminométhyl)-norbornane (BAMN) et la pipérazine (Pip).

Avantageusement, on a des blocs PA4.12, PA4.14, PA4.18, PA6.10, PA6.12, PA6.14, PA6.18, PA9.12, PA10.10, PA10.12, PA10.14 et PA10.18.

Selon un deuxième type, les blocs polyamides résultent de la condensation d'un ou plusieurs acides alpha oméga aminocarboxyliques et/ou d'un ou plusieurs lactames ayant de 6 à 12 atomes de carbone en présence d'un diacide carboxylique ayant de 4 à 12 atomes de carbone ou d'une diamine. A titre d'exemples de lactames, on peut citer le caprolactame, l'oenantholactame et le lauryllactame. A titre d'exemples d'acide alpha omega amino carboxylique, on peut citer les acides aminocaproïque, amino-7-heptanoïque, amino-11- undécanoïque et amino-12-dodécanoïque.

Avantageusement les blocs polyamides du deuxième type sont en polyamide 11, en polyamide 12 ou en polyamide 6.

Selon un troisième type, les blocs polyamides résultent de la condensation d'au moins un acide alpha oméga aminocarboxylique (ou un lactame), au moins une diamine et au moins un diacide carboxylique.

Dans ce cas, on prépare, les blocs polyamide PA par polycondensation :
- de la ou des diamines aliphatiques linéaires ou aromatiques ayant X atomes de carbone ;
- du ou des diacides carboxyliques ayant Y atomes de carbone ; et
- du ou des comonomères {Z}, choisis parmi les lactames et les acides alpha-oméga aminocarboxyliques ayant Z atomes de carbone et les mélanges équimolaires d'au moins une diamine ayant X1 atomes de carbone et d'au moins un diacide carboxylique ayant Y1 atomes de carbones, (X1, Y1) étant différent de (X, Y),
- ledit ou lesdits comonomères {Z} étant introduits dans une proportion pondérale allant jusqu'à 50%, de préférence jusqu'à 20%, encore plus avantageusement jusqu'à 10% par rapport à l'ensemble des monomères précurseurs de polyamide ;
- en présence d'un limiteur de chaîne choisi parmi les diacides carboxyliques ;

Avantageusement, on utilise comme limiteur de chaîne le diacide carboxylique ayant Y atomes de carbone, que l'on introduit en excès par rapport à la stoechiométrie de la ou des diamines.

Selon une variante de ce troisième type les blocs polyamides résultent de la condensation d'au moins deux acides alpha oméga aminocarboxyliques ou d'au moins deux lactames ayant de 6 à 12 atomes de carbone ou d'un lactame et d'un acide aminocarboxylique n'ayant pas le même nombre d'atomes de carbone en présence éventuelle d'un limiteur de chaîne. A titre d'exemple d'acide alpha omega amino carboxylique aliphatique, on peut citer les acides aminocaproïques, amino-7-heptanoïque, amino-11-undécanoïque et amino-12-dodécanoïque. A titre d'exemple de lactame, on peut citer le caprolactame, l'oenantholactame et le lauryllactame. A titre d'exemple de diamines aliphatiques, on peut citer l'hexaméthylènediamine, la dodécaméthylènediamine et la triméthylhexaméthylène diamine. A titre d'exemple de diacides cycloaliphatiques, on peut citer l'acide 1,4-cyclohexyldicarboxylique. A titre d'exemple de diacides aliphatiques, on peut citer les acides butane-dioïque, adipique, azélaïque, subérique, sébacique, dodécanedicarboxylique, les acides gras dimérisés (ces acides gras dimérisés ont de préférence une teneur en dimère d'au moins 98%; de préférence ils sont hydrogénés; ils sont commercialisés sous la marque "PRIPOL" par la société "UNICHEMA", ou sous la marque EMPOL par la société HENKEL) et les Polyoxyalkylènes - α,ω diacides. A titre d'exemple de diacides aromatiques, on peut citer les acides téréphtalique (T) et isophtalique (I). A titre d'exemple de diamines cycloaliphatiques, on peut citer les isomères des bis-(4-aminocyclohexyl)-méthane (BACM), bis-(3-méthyl-4-aminocyclohexyl)méthane (BMACM), et 2-2-bis-(3-méthyl-4-aminocyclohexyl)-propane(BMACP), et para-amino-di-cyclo-hexyl-méthane (PACM). Les autres diamines couramment utilisées peuvent être l'isophoronediamine (IPDA), la 2,6-bis-(aminométhyl)-norbornane (BAMN) et la pipérazine.

A titre d'exemples de blocs polyamides du troisième type, on peut citer les suivantes :
- 6.6/6 dans laquelle 6.6 désigne des motifs hexaméthylènediamine condensée avec l'acide adipique. 6 désigne des motifs résultant de la condensation du caprolactame.
- 6.6/6.10/11/12 dans laquelle 6.6 désigne l'hexaméthylènediamine condensée avec l'acide adipique. 6.10 désigne l'hexaméthylènediamine condensée avec l'acide sébacique. 11 désigne des motifs résultant de la condensation de l'acide aminoundécanoïque. 12 désigne des motifs résultant de la condensation du lauryllactame.

Les blocs polyéthers peuvent représenter 5 à 85 % en poids du copolymère à blocs polyamides et polyéthers. La masse Mn des blocs polyéther est comprise entre 100 et 6 000 g/mole et de préférence entre 200 et 3 000 g/mole.

Les blocs polyéthers sont constitués de motifs oxyde d'alkylène. Ces motifs peuvent être par exemple des motifs oxyde d'éthylène, des motifs oxyde de propylène ou tétrahydrofurane (qui conduit aux enchaînements polytétraméthylène glycol). On utilise ainsi des blocs PEG (polyethylène glycol) c'est à dire ceux constitués de motifs oxyde d'éthylène, des blocs PPG (propylène glycol) c'est à dire ceux constitués de motifs oxyde de propylène, des blocs PO3G (polytriméthylène glycol) c'est-à-dire ceux constitués de motifs polytriméthylène ether de glycol (de tels copolymères avec des blocs polytriméthylene ether sont décrits dans le document US6590065), et des blocs PTMG c'est à dire ceux constitués de motifs tetraméthylène glycols appelés aussi polytétrahydrofurane. Les copolymères PEBA peuvent comprendre dans leur chaîne plusieurs types de polyéthers, les copolyéthers pouvant être à blocs ou statistiques. De préférence, la composition selon l'invention comprend un PEBA dont les blocs polyéther sont composés majoritairement à base de PTMG, c'est-à-dire à plus de 50% en poids sur le poids total de blocs polyéther. En effet, il a été constaté de manière surprenante que ces PEBA base PTMG se solubilisaient très facilement dans le solvant selon l'invention. De préférence, le PEBA utilisé dans la composition de l'invention comprend plus de 50%, de préférence plus de 70%, voire plus de 75%, en poids de blocs polyéther sur le poids total de PEBA. En effet, il a été constaté que ces PEBA à fort pourcentage en poids de blocs polyéther étaient plus faciles à solubiliser que les PEBA à fort pourcentage en poids de blocs polyamide.

On peut également utiliser des blocs obtenus par oxyéthylation de bisphenols, tels que par exemple le bisphenol A. Ces derniers produits sont décrits dans le brevet EP613919.

Les blocs polyéthers peuvent aussi être constitués d'amines primaires éthoxylées. A titre d'exemple d'amines primaires éthoxylées on peut citer les produits de formule : 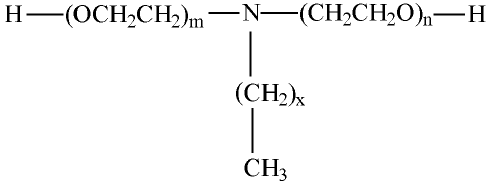 dans laquelle m et n sont compris entre 1 et 20 et x entre 8 et 18. Ces produits sont disponibles dans le commerce sous la marque NORAMOX® de la société CECA et sous la marque GENAMIN® de la société CLARIANT.

Les blocs souples polyéthers peuvent comprendre des blocs polyoxyalkylène à bouts de chaînes NH₂, de telles blocs pouvant être obtenues par cyanoacétylation de blocs polyoxyalkylène alpha-oméga dihydroxylés aliphatiques appelées polyétherdiols. Plus particulièrement, on pourra utiliser les Jeffamines (Par exemple Jeffamine® D400, D2000, ED 2003, XTJ 542, produits commerciaux de la société Huntsman, également décrites dans les documents de brevets JP2004346274, JP2004352794 et EP1482011).

Les blocs polyétherdiols sont soit utilisés tels quels et copolycondensés avec des blocs polyamides à extrémités carboxyliques, soit ils sont aminés pour être transformés en polyéther diamines et condensés avec des blocs polyamides à extrémités carboxyliques. La méthode générale de préparation en deux étapes des copolymères PEBA ayant des liaisons ester entre les blocs PA et les blocs PE est connue et est décrite, par exemple, dans le brevet français FR2846332. La méthode générale de préparation des copolymères PEBA de l'invention ayant des liaisons amide entre les blocs PA et les blocs PE est connue et décrite, par exemple dans le brevet européen EP1482011. Les blocs polyéther peuvent être aussi mélangés avec des précurseurs de polyamide et un limiteur de chaîne diacide pour faire les polymères à blocs polyamides et blocs polyéthers ayant des motifs répartis de façon statistique (procédé en une étape).

Bien entendu, la désignation PEBA dans la présente description de l'invention se rapporte aussi bien aux PEBAX® commercialisés par Arkema, aux Vestamid® commercialisés par Evonik®, aux Grilamid® commercialisés par EMS, qu'aux Kellaflex® commercialisés par DSM ou à tout autre PEBA d'autres fournisseurs.

Avantageusement, les copolymères PEBA ont des blocs PA en PA 6, en PA 11, en PA 12, PA 6.12, en PA 6.6/6, en PA 10.10 et/ou en PA 6.14, de préférence des blocs PA 11 et/ ou PA 12 ; et des blocs PE en PTMG, en PPG et/ou en PO3G. Les PEBAs à base de blocs PE constitués majoritairement de PEG sont à ranger dans la gamme des PEBAs hydrophiles. Les PEBAs à base de blocs PE constitués majoritairement de PTMG sont à ranger dans la gamme des PEBAs hydrophobes.

Avantageusement, ledit PEBA utilisé dans la composition selon l'invention est obtenu au moins partiellement à partir de matières premières bio-ressourcées.
Par matières premières d'origine renouvelable ou matières premières bio-ressourcées, on entend des matériaux qui comprennent du carbone bio-ressourcé ou carbone d'origine renouvelable. En effet, à la différence des matériaux issus de matières fossiles, les matériaux composés de matières premières renouvelables contiennent du ¹⁴C. La « teneur en carbone d'origine renouvelable » ou « teneur en carbone bio-ressourcé » est déterminée en application des normes ASTM D 6866 (ASTM D 6866-06) et ASTM D 7026 (ASTM D 7026-04). A titre d'exemple, les PEBA à base de polyamide 11 proviennent au moins en partie de matières premières bio-ressourcées et présentent une teneur en carbone bio-ressourcé d'au moins 1%, ce qui correspond à un ratio isotopique de ¹²C/¹⁴C d'au moins 1,2 x 10⁻¹⁴. De préférence, les PEBA selon l'invention comprennent au moins 50% en masse de carbone bio-ressourcé sur la masse totale de carbone, ce qui correspond à un ratio isotopique ¹²C/¹⁴C d'au moins 0,6.10⁻¹². Cette teneur est avantageusement plus élevée, notamment jusqu'à 100%, qui correspond à un ratio isotopique ¹²C/¹⁴C de 1,2 x 10⁻¹², dans le cas de PEBAs à blocs PA 11 et blocs PE comprenant du PO3G, PTMG et/ou PPG issus de matières premières d'orgine renouvelable.

Par alcool en C1 à C6 on entend les alcools dont le nombre de carbones de la chaîne carbonée ne dépasse pas 6, qui sont hydrosolubles, tels que l'éthanol, l'isopropanol. Des solutions alcooliques obtenues par simple mélange de ces alcools avec l'eau sont également utilisables dans la composition ; de même que les glycols, comme l'éthylène glycol, propylène glycol ; les polyols, comme le glycérol ou glycérine, le sorbitol, le sirop de sorbitol. On peut encore citer les butanediols 1,3 et 1,4 par exemple. En outre, les alcools aromatiques, tels que le métacrésol, l'alcool benzylique, peuvent être utilisés, mais sont moins préférés pour des raisons HSE.

Les alcools gras au sens de l'invention sont des alcools dont la chaîne carbonée comprend au moins 7 carbones, de préférence de 7 à 10 carbones, de sorte qu'ils sont liquides à température ambiante. La présence de l'hydroxyle leur confère une miscibilité avec l'alcool en C1 à C6. On peut notamment citer l'alcool benzylique qui joue le rôle de solvant et de conservateur.

Les acides gras au sens de l'invention sont des acides organiques qui se trouvent dans les lipides. Leur chaîne carbonée est plus ou moins longue (de C4 à C30) et ils peuvent être saturés ou insaturés. Les acides gras saturés sont solides à la température ambiante (25°C), sauf les acides en C4 et C6. Les acides gras insaturés sont liquides. A titre d'exemple, on peut citer l'acide laurique, l'acide stéarique, l'acide oléique.

Les esters gras résultent soit de la combinaison d'un acide gras avec un alcool à chaîne courte (par exemple le palmitate ou myristate d'isopropyle, le sébacate de diisopropyle, qui forment des esters gras liquides), soit de la combinaison d'un acide gras avec un alcool gras (par exemple l'isostéarate d'isostéaryle), soit de la combinaison d'un acide à chaîne courte avec un alcool gras à chaîne plus ou moins longue (par exemple acide benzoïque avec un alcool gras en C12-C15 formant des benzoates d'alcools gras, C12-C15 alkyl benzoate). A titre d'exemples d'esters gras utilisables dans les compositions selon l'invention, on peut egalement citer : les esters de l'acide benzoique, tels que le 2-Phényl ethyl ester de l'acide benzoïque, les esters de l'acide salicylique, tel que l'ethyl hexyl salicylate, les esters gras d'acide acrylique, tels que le 2-cyano-3.3-diphenylacrylate de 2-ethyl hexyle ; ou encore l'isopropyl lauryl sarcosinate, l'iso-nonanoate d'iso-nonyle, le dicaprylyl carbonate, etc.

Avantageusement, la composition selon l'invention comprend en outre de 1 à 10 parts d'additif choisi parmi : les filtres anti-UV, les antioxydants, pigments, charges comme le talc, nylon, silice, des actifs cosmétiques ou pharmaceutiques et tout autre agent pouvent également entrer dans la composition d'un revêtement, d'un vernis, d'un film souple transparent, d'un produit cosmétique ou pharmaceutique, pour améliorer la texture, l'étalement, le toucher, l'apparence, ou la stabilité de ladite composition.

La composition liquide selon l'invention est notamment fabriquée aisément par simple mélange, tel que mélange à reflux, du PEBA, dans l'éthanol et le co-solvant, de préférence en chauffant jusqu'à dissolution complète de tous les composants.

La présente invention a également pour objet l'utilisation de la composition selon l'invention pour la fabrication d'un produit ayant au moins l'une des formes suivantes : dispersion, solution, émulsion, microémulsion, nanoémulsion, émulsion sèche, suspension, aérosol, gel, notamment gel compact, gomme, gomme plastique, pâte, mousse, crème, poudre, telle que poudre libre, poudre compacte, poudre expansée, beurre, film, film élastique, et leurs mélanges ou associations.

La composition selon l'invention peut avantageusement être utilisée pour la fabrication d'objets résistants à l'eau, tels que des revêtements, des films, des vernis, des peintures, notamment des revêtements anti-graffitis, des joints, notamment des joints d'étanchéité, des équipements de protection individuelle, des gants, des masques souples, des préservatifs, des équipements protecteurs pour matériel électronique ou informatique, et toute autre application nécessitant un film souple étanche à l'eau.

Avantageusement, lesdits objets sont fabriqués par au moins une des méthodes suivantes : étalement, pulvérisation, sur un moule ou un support quelconque, notamment en bois, plastique, métal, la peau, les ongles, etc, par trempage, évaporation du solvant de la composition.

La composition selon l'invention peut notamment être utilisée pour la fabrication d'un produit cosmétique, pharmaceutique ou de parfumerie.

La présente invention a notamment pour objet une composition selon l'invention, telle que définie précédemment, ladite composition étant un produit coloré, non coloré et /ou transparent choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

### Exemples

Les exemples ci-dessous illustrent la présente invention sans en limiter la portée. Sauf indication contraire, tous les pourcentages et parts sont en poids.
PEBA 1 utilisé : copolymère à blocs PA12 et blocs PTMG de masses molaires respectives en g/mol (600 - 2000).
L'alcool utilisé est l'éthanol.
Les co-solvants suivants sont utilisés :
Co-solvant 1 : 2-cyano-3.3-diphenylacrylate de 2-ethyl hexyle
Co-solvant 2 : ethyl hexyl salicylate
Co-solvant 3 : isopropyl lauryl sarcosinate

Chacun de ces co-solvants est miscible avec l'éthanol.

### Comparatif 1 (sans co-solvant) :

Le PEBA1 (5 parts en poids) est solubilisé dans de l'éthanol (100 parts en poids) à chaud, 80°C. Cette solution se gélifie à froid, température comprise dans la gamme de 15 à 25°C.

### Comparatifs 2 (sans éthanol) :

Les trois co-solvants (1, 2 et 3) sont testés.

On disperse 5% en poids de 2533 dans chaque co-solvant pur, à l'ambiante (laisser agiter jusqu'à 24h au moins à température ambiante 15-25°C) ou en chauffant à 80°C (proche du point d'ébullition de l'éthanol) jusqu'à dissolution ou au maximum une journée.

Résultats de dissolution des granulés de PEBA 1 dans les différents solvants :

### - Co-solvants 2 et 3 :

Dans un flacon de 60 ml, on introduit 10g de solvant et 5% en poids de granulé de PEBA 1, soit 0.5g.

Le flacon est muni d'un barreau magnétique. On place le tout sur un agitateur magnétique sans chauffer.

Au bout de 8 heures d'agitation, les granulés ne se solubilisent pas.

### - Co-solvant 1 :

Ce solvant est trop visqueux pour pouvoir mettre une agitation, on modifie donc le mode opératoire. Dans ce cas on utilise un ballon de 250ml muni d'un réfrigérant et d'un barreau magnétique, le tout est placé sur un agitateur magnétique chauffant. On introduit 10 g de solvant et 5% de granulé PEBA1. On monte progressivement la température pour avoir un milieu agitable. Une température de 100°C est suffisante pour pouvoir agiter le milieu. Au bout de 5h00 les granulés sont dissous. Mais à froid (15-25°C) il y a la formation d'un gel.

Dans les essais suivants (tableau 1 : Cp3 et essais suivants), on travaille sous agitation (200 tr/min). On utilise un bain d'huile pour contrôler la température et un montage tube de verre classique (petit tube). On chauffe le mélange à reflux.

### Comparatif 3 avec le co-solvant 1 pur (Cp3) :

On met 20g de solvant dans le tube de verre additionné de 5% en poids de granulé PEBA 1 : soit 1g.
T=o on plonge le tube
T=20min les granulés commencent à gonfler, la température consigne est de 110°C.
T=240min on obtient un mélange gélatineux.

### Essais avec éthanol et co-solvant (Cp 4 à 11 et Ex 1 à 4) :

Dans ces essais, on utilise comme solvant un mélange co-solvant/éthanol. On teste différents ratios en volume. On additionne 5 parts en poids de PEBA 1 pour 100 parts de solvant total. La température consigne est de 80°C (point d'ébullition de l'éthanol). A partir d'un mélange à 60% de co-solvant, on augmente la température de 10°C pour solubiliser les granulés de PEBA 1.

Ces essais et leurs résultats sont rassemblés dans le tableau 1 suivant. Les colonnes sous « Co-solvant 1, 2 et 3 » indiquent la teneur en co-solvant (% en volume), sur le volume total de solvant, le reste du solvant étant l'éthanol.

### Résultats :

Pour obtenir une solution liquide stable à froid, c'est-à-dire à température ambiante (15-25°C), il est nécessaire d'utiliser au moins 15 (Cp6), voire 20% (Cp10) de co-solvant avec l'éthanol. Les co-solvants 1, 2 et 3, à partir de 51 % en volume dans le solvant total, donnent une solution liquide limpide à température ambiante.
D'après le tableau 1, on constate notamment que :
Pour 60% de co-solvant 1, on obtient une solution liquide limpide stable à température ambiante.
Pour 51% de co-solvant 2, on obtient une solution liquide limpide stable à température ambiante.
Pour 60% de co-solvant 3, on obtient une solution liquide limpide stable à température ambiante.
Pour 15-50% de co-solvant, la solution reste liquide à température ambiante mais est opaque.

**Tableau 1**

| Essai | Co-solvant 1 | Co-solvant 2 | Co-solvant 3 | Etat du produit à froid | Aspect du produit à froid |
|---|---|---|---|---|---|
| Cp3 | 100% | | | gel | opaque |
| Ex1 | 60% | | | liquide | limpide |
| Cp4 | 50% | | | Très visqueux | limpide |
| Cp5 | 40% | | | liquide | opaque |
| Ex2 | | 60% | | liquide | limpide |
| Ex3 | | 51% | | liquide | limpide |
| Cp6 | | 20% | | liquide | opaque |
| Cp7 | | 10% | | gel | opaque |
| Ex4 | | | 60% | Liquide visqueux | limpide |
| Cp8 | | | 50% | Liquide visqueux | opaque |
| Cp9 | | | 30% | liquide | opaque |
| Cp10 | | | 15% | liquide | opaque |
| Cp11 | | | 5% | gel | Très opaque |

Avec les essais des exemples conformes à la présente invention, Ex1, Ex2, Ex3, Ex4, on utilise le produit obtenu pour former un film. On étale une couche de produit sur un support quelconque : métal, bois, plastique, ongle, peau. On fait évaporer le solvant. On obtient un film, qui au contact de l'eau, résiste à l'eau et reste accroché au support.

## Revendications

1. Composition liquide comprenant de 1 à 15 parts en poids de PEBA pour 100 parts en poids de solvant, **caractérisée en ce que** :
ledit solvant comprend de 1 à 49% en volume d'alcool léger en C1 à C6 et de 51 à 99% en volume d'un cosolvant miscible avec l'alcool, sur le volume total de solvant,
le co-solvant est choisi parmi les acides gras, les esters gras, les alcools gras,
les éthers gras et leurs mélanges, et
la composition se présente sous la forme d'une solution limpide, présentant à 560 nm une transmission supérieure à 50%, à une température de 15 à 25°C.

2. Composition selon la revendication 1, **caractérisée en ce qu'**elle comprend de 1 à 10 parts, de préférence de 2 à 8 parts, de préférence de 4 à 6 parts de PEBA pour 100 parts de solvant.

3. Composition selon la revendication 1 ou 2, dans laquelle le PEBA comprend plus de 50%, de préférence plus de 70%, voire plus de 75%, en poids de blocs polyéther sur le poids total de PEBA.

4. Composition selon l'une quelconque des revendications 1 à 3, dans laquelle le PEBA comprend des blocs polyéther composés majoritairement à base de PTMG.

5. Composition selon l'une quelconque des revendications 1 à 4, **caractérisée en ce qu'**elle comprend en outre de 1 à 10 parts d'additif choisi parmi : les filtres anti-UV, les antioxydants, pigments, charges comme le talc, nylon, silice, des actifs cosmétiques ou pharmaceutiques.

6. Utilisation de la composition selon l'une quelconque des revendications 1 à 5 pour la fabrication d'un produit ayant au moins l'une des formes suivantes : dispersion, solution, émulsion, microémulsion, nanoémulsion, émulsion sèche, suspension, aérosol, gel, notamment gel compact, gomme, gomme plastique, pâte, mousse, crème, poudre, telle que poudre libre, poudre compacte, poudre expansée, beurre, film, film élastique, et leurs mélanges ou associations.

7. Utilisation selon la revendication 6 pour la fabrication d'objets résistants à l'eau, tels que des revêtements, des films, des vernis, des peintures, notamment des revêtements anti-graffitis, des joints, notamment des joints d'étanchéité, des équipements de protection individuelle, des gants, des masques souples, des préservatifs, des équipements protecteurs pour matériel électronique ou informatique.

8. Utilisation selon la revendication 6 ou 7 dans laquelle lesdits objets sont fabriqués par au moins une des méthodes suivantes étalement, pulvérisation, trempage, évaporation du solvant de la composition.

9. Utilisation selon la revendication 6 pour la fabrication d'un produit cosmétique, pharmaceutique ou de parfumerie.

10. Composition selon l'une quelconque des revendications 1 à 5, ladite composition étant un produit coloré, non coloré et /ou transparent, choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

## Patentansprüche

1. Flüssige Zusammensetzung, umfassend 1 bis 15 Gewichtsteile PEBA pro 100 Gewichtsteile Lösungsmittel, **dadurch gekennzeichnet, dass**:
das Lösungsmittel von 1 bis 49 Volumen-% leichten C1-bis C6-Alkohol und 51 bis 99 Volumen-% eines mit dem Alkohol mischbaren Co-Lösungsmittels, bezogen auf das Gesamtvolumen an Lösungsmittel, umfasst,
das Co-Lösungsmittel aus Fettsäuren, Fettsäureestern, Fettalkoholen, Fettethern und deren Gemischen ausgewählt ist und
die Zusammensetzung in Form einer durchsichtigen Lösung vorliegt, die bei 560 nm eine Transmission von mehr als 50% bei einer Temperatur von 15 bis 25°C aufweist.

2. Zusammensetzung nach Anspruch 1, **dadurch gekennzeichnet, dass** sie 1 bis 10 Teile, vorzugsweise 2 bis 8 Teile, bevorzugt 4 bis 6 Teile PEBA pro 100 Teile Lösungsmittel aufweist.

3. Zusammensetzung nach Anspruch 1 oder 2, wobei das PEBA mehr als 50 Gew.-%, vorzugsweise mehr als 70 Gew.-%, sogar mehr als 75 Gew.-% Polyetherblöcke, bezogen auf das Gesamtgewicht an PEBA, aufweist.

4. Zusammensetzung nach einem der Ansprüche 1 bis 3, wobei das PEBA Polyetherblöcke umfasst, deren Zusammensetzung größtenteils auf PTMG basiert.

5. Zusammensetzung nach einem der Ansprüche 1 bis 4, **dadurch gekennzeichnet, dass** sie außerdem 1 bis 10 Teile Zusatzstoff, ausgewählt aus: UV-Filtern, Antioxidantien, Pigmenten, Füllstoffen, wie Talk, Nylon, Siliziumdioxid, kosmetischen oder pharmazeutischen Wirkstoffen, umfasst.

6. Verwendung der Zusammensetzung nach einem der Ansprüche 1 bis 5 zur Herstellung eines Produkts, das mindestens eine der folgenden Formen besitzt: Dispersion, Lösung, Emulsion, Mikroemulsion, Nanoemulsion, trockene Emulsion, Suspension, Aerosol, Gel, insbesondere Kompaktgel, Gummi, Kunststoffgummi, Paste, Schaum, Creme, Puder, wie loser Puder, Kompaktpuder, expandierter Puder, Butter, Film, elastischer Film und deren Gemische oder Vermengungen.

7. Verwendung nach Anspruch 6 zur Herstellung von wasserbeständigen Gegenständen, wie Beschichtungen, Filme, Lacke, Anstriche, insbesondere Anti-Graffiti-Beschichtungen, Verbindungen, insbesondere Dichtungsverbindungen, persönliche Schutzausrüstungen, Handschuhe, anpassungsfähige Masken, Präservative, Schutzausrüstungen für elektronische oder Informatikgeräte.

8. Verwendung nach Anspruch 6 oder 7, wobei die Gegenstände durch mindestens eines der folgenden Verfahren hergestellt werden: Verteilen, Zerstäubung, Eintauchen, Verdampfen des Lösungsmittels der Zusammensetzung.

9. Verwendung nach Anspruch 6 zur Herstellung eines kosmetischen, pharmazeutischen oder Parfümerieprodukts.

10. Zusammensetzung nach einem der Ansprüche 1 bis 5, wobei die Zusammensetzung ein gefärbtes, farbloses und/oder transparentes Produkt ist, das aus den folgenden Produkten ausgewählt ist:
- Schminkprodukten für das menschliche Gesicht und den menschlichen Körper, wie Makeup-Grundierung, getönte Creme, loser oder Kompaktpuder, Lidschatten, Wimperntusche, Eyeliner, Lippenstift, Nagellack;
- Pflegeprodukten für das menschliche Gesicht und den menschlichen Körper, wie Creme, Milch, Lotion, Maske, Peelingprodukte, Reinigungs- und/oder Abschminkprodukte, Deodorants, Antitranspirants, Antiperspirants, Rasierprodukte, Epilierprodukte;
- Haarprodukten, wie Shampoos, Produkte für das Formen der Haare, Produkte zur Erhaltung der Frisur, Antischuppenprodukte, Produkte gegen Haarausfall, Produkte gegen Haartrockenheit, Haarfärbemittel, Entfärbungsprodukte;
- Parfümerieprodukten, wie Parfum, Milch, Creme, parfümierter loser oder Kompaktpuder.

## Claims

1. Liquid composition comprising from 1 to 15 parts by weight of PEBA per 100 parts by weight of solvent, **characterized in that**:
said solvent comprises from 1% to 49% by volume of light C₁ to C₆ alcohol and from 51% to 99% by volume of a cosolvent which is miscible with the alcohol, with respect to the total volume of solvent,
the cosolvent is chosen from fatty acids, fatty esters, fatty alcohols, fatty ethers and their mixtures, and the composition is provided in the form of a solution which is clear, exhibiting, at 560 nm, a transmission of greater than 50%, at a temperature of 15 to 25°C.

2. Composition according to Claim 1, **characterized in that** it comprises from 1 to 10 parts, preferably from 2 to 8 pars, preferably from 4 to 6 parts, of PEBA, per 100 parts of solvent.

3. Composition according to Claim 1 or 2, in which the PEBA comprises more than 50% by weight, preferably more than 70% by weight, indeed even more than 75% by weight, of polyether blocks with respect to the total weight of PEBA.

4. Composition according to Claims 1 to 3, wherein the PEBA comprises polyether blocks composed predominantly on the basis of PTMG.

5. Composition according to any one of Claims 1 to 4, **characterized in that** it additionally comprises from 1 to 10 parts of additive chosen from: UV screening agents, antioxidants, pigments, fillers, such as talc, nylon or silica, or cosmetic or pharmaceutical active agents.

6. Use of the composition according to any one of Claims 1 to 5 in the manufacture of a product having at least one of the following forms: dispersion, solution, emulsion, microemulsion, nanoemulsion, dry emulsion, suspension, aerosol, gel, in particular compact gel, gum, plastic gum, paste, foam, cream, powder, such as loose powder, compact powder or expanded powder, butter, film, elastic film and their mixtures or combinations.

7. Use according to Claim 6 in the manufacture of water-resistant objects, such as coatings, films, varnishes, paints, in particular antigraffiti coatings, seals, in particular leaktight seals, individual protective equipment, gloves, flexible masks, condoms or protective accessories for electronic or computing equipment.

8. Use according to Claim 6 or 7, wherein said objects are manufactured by at least one of the following methods: spreading, spraying, dipping, evaporation of the solvent from the composition.

9. Use according to Claim 6 in the manufacture of a cosmetic, pharmaceutical or perfumery product.

10. Composition according to any one of Claims 1 to 5, said composition being a colored, colorless and/or transparent product chosen from the following products:
- makeup products for the human face and body, such as foundation, tinted cream, loose or compact powder, eyeshadow, mascara, eyeliner, lipstick or nail varnish;
- care products for the human face and body, such as cream, milk, lotion, mask, scrubbing product, cleansing and/or makeup-removing products, deodorants, antiperspirants, shaving products or hair-removing products;
- hair products, such as shampoos, products for the shaping of the hair, products for retaining the hairstyle, antidandruff products, products for combating hair loss, products for combating dryness of the hair, hair dyes or bleaching products;
- perfumery products, such as fragrance, milk, cream, or loose or compact scented powder.
